# EUROPEAN PATENT SPECIFICATION

(11) **EP 4 096 416 B1**
(45) Date of publication and mention of the grant of the patent: **13.03.2024**
(21) Application number: 21703173.1
(22) Date of filing: 28.01.2021
(51) Int. Cl.: A23C 19/06, A23L 11/50, A23C 9/12, A23C 9/123, A23C 11/10, C12P 39/00

(54) **A METHOD FOR DETERMINING AN ORDER IN WHICH A FOOD PRODUCT IS CULTURED**
VERFAHREN UND SYSTEM ZUM BESTIMMEN EINER REIHENFOLGE, IN DER EIN LEBENSMITTELPRODUKT NACHEINANDER MIT EINEM SATZ VON BAKTERIENKULTUREN KULTIVIERT WIRD
PROCÉDÉ ET SYSTÈME POUR DÉTERMINER UN ORDRE DANS LEQUEL UN PRODUIT ALIMENTAIRE EST CULTIVÉ SUCCESSIVEMENT AVEC UN ENSEMBLE DE CULTURES BACTÉRIENNES

(30) Priority: 30.01.2020 EP 20154681
(43) Date of publication of application: 07.12.2022
(60) Divisional application: 24155457.5
(73) Proprietor: DSM IP Assets B.V., 6411 TE Heerlen (NL)
(72) Inventor: VAN HEE, Pim, 6100 AA Echt (NL)
(74) Representative: dsm-firmenich IP
(86) International application number: PCT/EP2021/051925
(87) International publication number: WO 2021/151978

(56) References cited:
- EP-A2- 0 474 463
- EP-A2- 1 466 985
- GB-A- 2 419 601
- US-A1- 2017 096 635
- DURMAZ E ET AL: "A Starter Culture Rotation Strategy Incorporating Paired Restriction/ Modification and Abortive Infection Bacteriophage Defenses in a Single Lactococcus lactis Strain.", APPLIED AND ENVIRONMENTAL MICROBIOLOGY, vol. 61, no. 4, 1 January 1995 (1995-01-01), pages 1266-1273, XP055800339, US ISSN: 0099-2240, DOI: 10.1128/AEM.61.4.1266-1273.1995 Retrieved from the Internet: URL:https://aem.asm.org/content/aem/61/4/1 266.full-text.pdf> [retrieved on 2021-04-30]

## Description

### Field of the invention

The invention relates to a method for determining an order in which a food product is successively cultured with a set of bacterial cultures. The invention further relates to a process for producing a fermented food product by successively culturing a food product with a set of bacterial cultures. Furthermore, the invention relates to a computer program product for selecting consecutive bacterial cultures for culturing a food product in a process for producing a fermented food product.

### Background of the invention

Fermentation in food processing is a process wherein carbohydrates are converted into organic acids or alcohol, with the use of microorganisms under anaerobic conditions. Fermentation is often performed with yeasts or bacteria as microorganisms. Almost any food product can be fermented, such as milk, olives, beans, grains, fruit such as grapes, honey, other dairy products, fish, meat and tea.

A variety of bacterial genera are used for fermentation, for example *Streptococcus, Acetobacter, Bacillus, Bifidobacterium, Lactobacillus* etc. *Lactobacillus* for example, is able to convert sugars into lactic acid, and therefore actively lowering the pH of its environment. Some *Lactobacillus* species can be used as starter cultures for a high variety of fermented products, such as yogurt, cheese, sauerkraut, pickles, beer, cider, kimchi, cocoa, kefir and other fermented foods.

Bacteria that are used for fermentation can be prone to bacteriophages. A bacteriophage, or just phage, is a virus that can infect and replicate within bacteria and archaea. Phages are very diverse and common, and for virtually each bacterial strain at least one phage exists that can infect said bacterial strain. Nevertheless, phages are often only able to infect and divide within a small subset of bacterial strains. The latter can be explained by either antagonistic pleiotropy, wherein adaptation to one host is advantageous but might be deleterious in another host, or when a more general mechanism of infection is chosen, efficiency is reduced. Phages can be beneficially employed as an antibacterial agent, such as in phage therapy, and have many potential applications in human medicine as well as dentistry, veterinary science, and agriculture. Because of the specificity of phages, very specific treatments can be devised.

However, bacteriophages can also cause detrimental effects. This is the case in, for example the dairy industry, wherein the bacteriophages can inhibit fermentation of dairy by bacteria. Bacterial fermentation to produce other fermentation products can suffer from the same consequences. An example is the fermentation of soybean with *Bacillus subtilis.* Because of the specificity of bacteriophages, it is possible to change one bacterial strain for another to resolve bacteriophage infection. However, this often leads to suboptimal process for which the output is difficult to predict. EP0474463 discloses a bacterial rotation strategy for use in successive fermentation of a food product by fermenting the substrate with a bacterial culture carrying a first known phage defense mechanism and thereafter incubating the substrate with a second or further bacterial strain, wherein the second or further strain carries a second or further phage defense mechanism different from the first phage defense mechanism.

There is thus a need for bacterial rotation schemes in order to reduce the influence of bacteriophages, even when each bacterial strain is not characterized and/or known.

### Summary of the invention

It is an object of the invention to provide for a method and a system that obviates at least one of the above mentioned drawbacks.

Additionally or alternatively, it is an object of the invention to determine an order with which a food product is successively cultured with bacteria sensitive to bacteriophages.

Additionally or alternatively, it is an object of the invention to dynamically change bacterial strains during bacterial fermention to reduce the bacteriophage infection grades.

Thereto, the invention provides for a method for determining an order in which a food product is successively cultured with a set of bacterial cultures in a process for producing a fermented food product. The set of bacterial cultures comprises a first subset of one or more bacterial cultures comprising one or more unknown bacterial strain, and a second subset of one or more bacterial cultures comprising one or more known bacterial strains, wherein each of the bacterial strains of the second subset is known. The process is carried out with an initial order of the set of bacterial cultures. During culturing with each bacterial culture of the first subset a process sample is collected. A culture sample of a bacterial culture of the second subset is exposed to the collected process samples in order to determine bacteriophage sensitivities of the one or more bacterial cultures of the second subset to bacteriophages present in the collected process sample. An adapted order of the set of bacterial cultures is determined based on the determined bacteriophage sensitivities of each of the cultures, such as to reduce common bacteriophage sensitivities in successive bacterial cultures.

A great advantage of this method is that unknown bacterial cultures can easily be mixed in between known bacterial cultures, without elaborate testing. This results in an earlier detection of possible harmful bacteriophage infections. As a consequence, financial losses are less frequent and less food products, such as milk, are being wasted.

In some examples, the process is performed for producing a milk-based product such as cheese. Such a process may involve a multiple number of phage rotations. There is a desire to determine an improved phage rotation order, even when data about some bacterial cultures employed in the process is unknown. The initial order may have at least one bacterial culture with unknown data, e.g. a bacterial culture from a third party such as a competitor with limited available data. This can for example be the case when cultures are bought of multiple culture suppliers, where each culture supplier only has transparency on their own cultures. The predetermined rotation scheme can be analyzed and an adjustment of the initial order can be proposed based on determined bacteriophage sensitivities. The bacteriophage sensitivities can be determined from sample analysis.

By not having to test each bacterial strain from each bacterial culture to each other bacterial culture, less time is lost and an adapted order of bacterial cultures can be decided upon much earlier.

The term "food product", as used herein, may refer to any substance that can be used or prepared for use as food. Food is a substance that can be eaten by an animal, such as a human, and often comprises carbohydrates, fats, proteins and/or water.

The term "unknown bacterial culture", as used herein, may refer to a bacterial culture comprising at least one unknown bacterial strain. The term "unknown bacterial strain", as used herein, may refer to a bacterial strain that has not been identified, e.g. of which the genus, species and/or strain, and/or nucleic acid sequence, and/or bacteriophage sensitivity is unknown.

The term "known bacterial culture", as used herein, may refer to a bacterial culture wherein all bacterial strains are known bacterial strains. The term "known bacterial strain", as used herein, may refer to any bacterial strain which is identified, e.g. of which the genus, species and/or strain, and/or nucleic acid sequence and/or bacteriophage sensitivity are known.

In some examples, a food producer may use bacterial cultures coming from two or more providers, such as for instance bacterial culture providers. The food producer may employ a mix of the bacterial cultures of the different providers. From the viewpoint of the food producer and/or one of the providers, at least some of the bacterial cultures provided by one or more of the (other) providers may be unknown. For instance, from the viewpoint of a first provider, known bacterial cultures are provided, and the other provider(s) may provide bacterial cultures which are unknown or for which no information is available for adequate assessment of the bacteriophage sensitivity. The first provider of bacterial cultures may provide the food producer with a correct order of the provided bacterial cultures, as the first provider would have tested each culture against known bacteriophages and each other.

In a first step the food producer may ferment the food product with each of the bacterial cultures at a time, while taking a process sample each time a bacterial culture is used of a different provider (e.g. a provider who wants to keep silent with regard to detailed information regarding the bacterial culture and/or its sensitivity to bacteriophages). These process samples can then be tested against the bacterial cultures of the first provider, and bacteriophage growth can be evaluated in each of these cultures for each of the samples. When bacteriophages can grow in the known bacterial culture, it indicates that the unknown bacterial culture related to the sample has a common bacteriophage sensitivity with the known bacterial culture. Lastly, the first provider can determine an adapted order wherein the common bacteriophage sensitivities are reduced in successive bacterial cultures. Hence, in view of the above, the adapted order can be obtained even when detailed information of a subgroup of the bacterial cultures used are unknown for being able to assess its bacteriophage sensitivity. Even in a case wherein a mix of known and unknown bacterial cultures (to the party wanting to optimize the order) is used in the process, one party can determine how the order is to be adapted in order to reduce the risk of a failed process due to bacteriophage infections.

The terms "bacteriophage", "phage" and the like, as used herein, may refer to a virus that is only able to infect and replicate within bacteria and archaea. Bacteriophages are composed of proteins that encapsulate a DNA or RNA genome, and often have a typical outlook of an icosahedral envelop head encapsulating the nucleic acid with a tail, made up of a sheath and a baseplate with fibers attached. The tail will attach to the bacterium or the archaea, and the nucleic acid is inserted via there. The host-microorganism is then forced to translate the DNA or RNA from the bacteriophage into bacteriophage components. After assembly, the host, e.g. the bacterium, is forced to release the phages and the bacterium is often destroyed in the process.

Bacteriophages are present everywhere, including in bulk starter cultures. Generally, bacterial cultures are rotated during the process, in the hope that the strains in the cultures are different so no common bacteriophage sensitivities are present.

The terms "sensitivity", "bacteriophage sensitivity", "phage sensitivity" and the like, as used herein, may refer to the ability of a bacterial culture or a bacterial strain to be infected by a specific bacteriophage. When bacterial cultures or bacterial strains are said to have a common bacteriophage sensitivity, or the like, it may refer to said bacterial cultures or bacterial strains being able to be attacked or infected by at least one bacteriophage, but not necessarily all bacteriophages present in the bacterial cultures or bacterial strains. A bacteriophage attack or bacteriophage infection comprises the insertion of bacteriophage DNA or RNA into its host, here bacteria. Additionally, the bacteriophage DNA or RNA is replicated and translated by the bacterium, resulting in a large amount of said bacteriophage. These are then released to the environment, able to infect or attack other bacteria.

The term "rotation", as used herein, may refer to a cycle wherein each bacterial culture is used once in the fermentation process before starting a new process cycle. This invention optimizes the order of bacterial cultures in the rotation of bacterial cultures.

In some examples, a food producer obtains bacterial cultures of more than one provider. A first provider of bacterial cultures will provide the food producer with the correct order of his provided bacterial cultures, as the first provider would have tested each culture against known bacteriophages and each other. However, whenever a food producer wants to mix bacterial cultures in his rotation scheme of more than one provider, he does not know the correct rotation order of each of the bacterial cultures, as providers of bacterial cultures usually do not disclose bacteriophage information of bacterial cultures. If bacterial cultures of different providers are used, the food producer has no way to optimize the order but trial and error, or characterizing each bacterial culture. However, with the above-mentioned method, this is no longer necessary. By running the rotation of bacterial cultures just once, analysis can be performed by one of the providers on a sample resulting from the bacterial cultures that comprise unknown strains.

The process is preferably carried out with an initial order of the set of bacterial cultures with a sample of the food product.

Optionally, in a subsequent process the food product, or a sample thereof, is successively cultured with the set of bacterial cultures in the adapted order.

By establishing an adapted order wherein none of the successive bacterial cultures have common bacteriophage sensitivities, the subsequent process will be more phage robust and thereby less production time is lost and less food product needs to be downgraded.

Optionally, the adapted order is employed dynamically during the process for producing the fermented food product.

The term "employed dynamically", as used herein, may refer to adapting the order during the process, and not necessarily waiting until the end of the rotation of bacterial cultures. By taking samples and analyzing these during the process, not waiting for the whole rotation process to finish, it would be possible to detect possible bacteriophage sensitivities and thus bacteriophage infections at an earlier time point. This makes the optimization process faster and the fermentation phage robust in a shorter time span.

Optionally, each bacterial strain of the one or more bacterial cultures of the second subset is exposed to the collected process sample for determining the bacteriophage sensitivities.

By testing each of the bacterial strains of the known bacterial cultures, it would be possible to actually identify bacteriophage sensitivities in the bacterial cultures of the first subset, while still performing only a minimal amount of experiments. By doing these additional tests and identifying bacterial strains present in the unknown bacterial cultures, additional known cultures can be added to the rotation, without the need to test them for common bacteriophage sensitivities with the formerly unknown bacterial cultures, if the common bacteriophage sensitivities of this new known culture compared with the other known used cultures, is known.

Optionally, one or more bacterial strains of the one or more bacterial cultures of the second subset are individually exposed to each of the collected process samples.

It can be useful to identify a specific bacteriophage sensitivity within the first subset of bacterial cultures. One way to achieve this is to test each of the process samples of the first subset against a specific bacterial strain comprised in the second subset. This can be performed for one or more bacterial strain of the second subset separately.

Optionally, combined bacterial strains of the one or more bacterial cultures of the second subset are exposed to each of the collected process samples.

To reduce time restraints, a quick check whetherthe known and unknown bacterial cultures are compatible regarding bacteriophage sensitivity can be performed. In this case, bacterial strains from the known bacterial cultures can be grouped, e.g. splitting each bacterial culture in two or more parts, each part containing at least one bacterial strain.

Furthermore, every bacterial culture can be checked in their entirety for compatibility with the unknown bacterial cultures, representing the quickest way to establish common phage sensitivities.

Optionally, a process sample is collected during or after culturing with each bacterial culture of the first and second subset.

Optionally, the adapted order of the set of bacterial cultures is selected such as to minimize common sensitivities to bacteriophages in successive bacterial cultures.

Even when common phage sensitivities are found between unknown and known bacterial cultures, it is still possible to minimize bacteriophage infection risk by minimizing the common bacteriophage sensitivities in subsequent fermentations. When a bacterial culture comprises more than one bacterial strain, these bacterial strains are present in a certain proportion. To minimize common bacteriophage sensitivities, and thus optimize the process, common bacteriophage sensitivities of bacterial strains in high proportion in the bacterial culture should be avoided in consecutive bacterial cultures. When it is not possible to remove all common bacteriophage sensitivities in consecutive bacterial cultures, common bacteriophage sensitivities of bacterial strains in low proportion in consecutive bacterial cultures can be chosen. A model can be used to solve this optimization problem.

Optionally, determining bacteriophage sensitivities is performed by determining a value indicative for the number of bacteriophages in the collected process sample.

By quantifying the bacteriophages, it would be possible to quantify the risk on infection when it is preceded or followed by a bacterial culture sensitive to said identified and/or quantified bacteriophage. The term "number of bacteriophages", as used herein, may refer to the amount of individual bacteriophages, regardless of the type of bacteriophages. It is thus a method to quantify the bacteriophages and estimate the sensitivity of the bacterial cultures and bacterial strains of the set.

Optionally, the value indicative for the number of bacteriophages is determined by performing one or more quantitative polymerase chain reaction (qPCR) measurements.

Quantitative polymerase chain reaction (qPCR) has as advantage that it is a very quick method, thus resulting in an even faster method for detecting bacteriophage sensitivities. qPCR is also often known as real-time polymerase chain reaction (real-time PCR). Optionally, other quantitative DNA detection methods can be used for determining the value, preferably with a DNA amplification method. PCR is widespread method for exponentially amplifying DNA sequences. This is performed by thermal cycling: when the temperature is high, the DNA strain is split into two single-stranded DNA molecules due to the breaking of hydrogen bonds between the complementary bases. When the temperature is lowered , primers are able to bind to the single-stranded DNA, resulting of annealing of the primers to the single-stranded DNA. These primers are chosen specifically for which DNA sequence needs to be multiplied. In a third part of the thermal cycle, the complementary part of the single strand DNA is added by elongating the primer, by providing the mixture with a DNA polymerase, for example *Thermus aquaticus* (*Taq*) polymerase, and free deoxyribonucleotides (dNTPs), which are to be inserted. This last step is carried out at a temperature suitable for the DNA polymerase, e.g. for *Taq* polymerase. The cycle is started anew after this last step. Usually, this is repeated for about 20-40 times.

In qPCR, the reaction is followed during the thermal cycling, for example via the addition of a non-specific fluorescent dye that is able to intercalate with any double-stranded DNA, or via the use of sequence-specific DNA probes consisting of oligonucleotides that have been labeled with a fluorescent reporter. The latter option is only detectable after hybridization with its complementary sequence.

Optionally, the value indicative for the number of bacteriophages is determined by performing one or more isothermal amplification methods, e.g. Recombinase Polymerase Amplification (RPA) or loop-mediated isothermal amplification. These methods are similar to PCR, but do not require the changing of temperature during the cycle.

Optionally, qPCR is used to detect and quantify bacteriophage DNA present in the sample.

Optionally, the fermented food product is a dairy product, preferably a cheese or a yoghurt.

Bacteriophages are a large hazard in the fermentation of milk, as they can infect the bacteria that are used for the acidification of milk in the production of fermented milk products, such as yoghurt, sour milk, quark, cream cheese, soft cheese, semi-soft cheese, semi-hard cheese, hard cheese, soured cream, cultured butter, sour cream, crème fraiche, mascarpone, sour milk cheese, buttermilk, schmand or smetana, blue vein cheese, etc.

This acidification can be performed with a wide range of bacteria, such as *Lactococcus lactis* ssp. *lactis* and *Lactococcus lactis* ssp. *cremoris, Streptococcus salivarius* ssp. *thermophiles, Lactobacillus helveticus, Propionibacter shermani,* etc.

Milk can be from an animal source, e.g. cow, goat, sheep, buffalo, etc. Additionally, milk can also have a non-dairy source, such as plant milk. Examples of plant milk include almond milk, coconut milk, rice milk, soy milk, etc.

Optionally, determining the value indicative for the number of bacteriophages is performed by detecting and/or identifying bacteriophages in the sample of the process for producing a fermented food product.

By detecting and/or identifying the bacteriophages in the process sample, the value indicative for the number of bacteriophages can easily be calculated. This value can then be used to determine the bacteriophage sensitivities of the bacterial culture or bacterial culture.

Optionally, information regarding bacteriophage sensitivities are stored in a database, wherein the database is used for determining the culturing order in a different process for producing fermented food product.

By creating a database, it will be easier to create a phage-robust rotation for later or adapted processes for producing fermented food products. The database comprises information regarding bacteriophage sensitivities, for example to which bacteriophages, which other bacterial cultures or bacterial strains have common bacteriophage sensitivities, the value indicative for the number of bacteriophages in the bacterial culture or bacterial strain in orderto determine the growth rate of the bacteriophages in said bacterial culture or bacterial strain, etc.

Optionally, the set of bacterial cultures includes two to twenty bacterial cultures, more preferably three to ten bacterial cultures, even more preferably three to six bacterial cultures

The more different bacterial cultures are used, the more variation exists in bacteriophage sensitivity if the bacterial cultures do not have any common bacteriophage sensitivities in successive bacterial cultures, thus the more phage robust the rotation. However, it is also important to take into account the continuity of the characteristics of the resulting fermented food product, which should be the same for each bacterial culture that is used in the process. Therefore, a more optimal amount of bacterial cultures is three, four or five bacterial cultures. In this case, the characteristics of the resulting fermented food product should be conserved, while still providing a robust phage rotation, as opposed to only one or two bacterial cultures.

Optionally, the one or more bacterial cultures of the first subset are provided by one or more first providers, and wherein the one or more bacterial cultures of the second subset are provided by one or more second providers, wherein the one or more first and second providers are different.

In this way, the one or more second supplier has knowledge of all the known bacterial cultures, and is able to carry out the step of exposing the culture samples to the process samples, as he would have the required culture samples.

According to an aspect, the invention provides for a process for producing a fermented food product by successively culturing a food product with a set of bacterial cultures, wherein an order of bacterial cultures in which the food product is successively cultured is determined by performing a method for determining an order in which a food product is successively cultured with a set of bacterial cultures in a process for producing a fermented food product. Each bacterial culture of the set of bacterial cultures includes at least one bacterial strain, wherein the set of bacterial cultures comprises a first subset of one or more bacterial cultures comprising one or more unknown bacterial strain, and a second subset of one or more bacterial cultures comprising one or more known bacterial strains, wherein each of the bacterial strains of the second subset is known. The process is carried out with an initial order of the set of bacterial cultures. During culturing with each bacterial culture of the first subset a process sample is collected. A culture sample of a bacterial culture of the second subset is exposed to the collected process samples in order to determine bacteriophage sensitivities of the one or more bacterial cultures of the second subset to bacteriophages present in the collected process sample. An adapted order of the set of bacterial cultures is determined based on the determined bacteriophage sensitivities such as to reduce common bacteriophage sensitivities in successive bacterial cultures.

In an example, a cheese is produced employing 4 phage rotations, e.g. rotation scheme A-B-C-D. A problem may arise when two (or more) suppliers deliver their own rotation schemes and the food producer starts using them. Then the rotation schemes are no longer aligned and optimally chosen, which may often occur in practice. The invention provides a way to determine how the rotation scheme (adapted order of bacterial cultures) can be optimized using rotations from multiple providers (e.g. parties, suppliers, competitors, etc.).

In some examples, whey samples are taken from the process, which are then analyzed in relation to known rotations (cf. bringing the samples in contact with known bacterial cultures and/or strains of known bacterial cultures). From the results, it can be determined what the effect of the whey samples is on the known strains. The bacteriophage sensitivities can be determined and based on that a proposed modified rotation scheme can be determined. Each rotation may have one or more bacterial cultures, each bacterial culture having at least one strain. The bacterial culture may also have a group of a plurality of bacterial strains.

The bacteriophage sensitivities can be assessed based on their overlapping sensitivities. Preferably there is no overlap. However, in some cases there is partial overlap, so there should be as little as possible overlap in phage sensitivity for the bacterial cultures or its strains. It is possible to select a most distance neighbor based on the available bacteriophage sensitivity information (cf. based on overlap in sensitivity).

In the adapted order, successive bacterial cultures can be chosen such that the one or more bacterial strains of the successive bacterial cultures have the least bacteriophage sensitivity overlap.

Also, provided is a system for determining an order in which a food product is successively cultured with a set of bacterial cultures in a process for producing a fermented food product, each bacterial culture of the set of cultures including at least one bacterial strain, wherein the set of bacterial cultures includes a first subset of one or more bacterial cultures having at least one unknown bacterial strain, and a second subset of one or more bacterial cultures with one or more known bacterial strains; wherein each of the bacterial strains of the second subset is known; wherein the system is configured to carry out the process with an initial order of the set of bacterial cultures; wherein the system includes a sampling unit configured to collect a process sample during culturing with each bacterial culture of the first subset; wherein the system is configured to expose a culture sample of one or more bacterial culture of the second subset to the collected process sample, wherein the system further includes a sensory unit adapted to determine bacteriophage sensitivities of the one or more bacterial cultures of the second subset to bacteriophages present in the one or more collected process sample; and wherein the system is configured to determine, by means of a controller, an adapted order of the set of bacterial cultures based on the determined bacteriophage sensitivities for reducing common bacteriophage sensitivities in successive bacterial cultures.

Optionally, the system can include a phage test kit that quantifies the phages in the process sample instead of exposing the process sample to the bacterial cultures of the second subset.

Advantageously, an improved bacterial culture rotation scheme employed in the process can be determined. The rotation scheme can be optimized, not only using known bacterial cultures, but also unknown bacterial cultures (e.g. with limited information from a third party such as another provider). Hence, the bacterial cultures employed in the process are only partially known, more specifically only a subgroup of the bacterial cultures are known. For instance, a food producer may have a process at a plant using different phage rotations (R1, R2, R3, R4). The bacterial cultures may be used in a specific order initially. In this example R1 and R2 have a first provider, and R3 and R4 have a second provider, different from the first provider. By analyzing whey samples from that process against the bacterial cultures (and/or strains thereof) from the first provider of R1 and R2, a relationship between the phage sensitivities of the R3 and R4 rotations (second provider) and R1 and R2 rotations can be determined. Once the sensitivities between R1, R2, R3 and R4 are determined, an order of the rotations can be adapted.

According to an aspect, the invention provides for a computer program product configured to be run on a machine for selecting consecutive bacterial cultures for culturing a food product in a process for producing a fermented food product, the computer program product being configured to perform the method for determining an order in which a food product is successively cultured with a set of bacterial cultures in a process for producing a fermented food product. Each bacterial culture of the set of bacterial cultures includes at least one bacterial strain, wherein the set of bacterial cultures comprises a first subset of one or more bacterial cultures comprising one or more unknown bacterial strain, and a second subset of one or more bacterial cultures comprising one or more known bacterial strains, wherein each of the bacterial strains of the second subset is known. The process is carried out with an initial order of the set of bacterial cultures. During culturing with each bacterial culture of the first subset a process sample is collected. A culture sample of a bacterial culture of the second subset is exposed to the collected process samples in order to determine bacteriophage sensitivities of the one or more bacterial cultures of the second subset to bacteriophages present in the collected process sample. An adapted order of the set of bacterial cultures is determined based on the determined bacteriophage sensitivities such as to reduce common bacteriophage sensitivities in successive bacterial cultures.

In an example, a food producer employs a process with a scheme having bacterial cultures (rotations) in the following order: R3, R1, R4, R2. R1 and R2 are known and R3 and R4 are unknown (lacking or missing information). First, the rotation of R3 can be employed. Then, (whey) samples can be taken from that process step. The (whey) samples can come from the process in which R3 was used. These can be analyzed against the R1 and R2 rotations. The (whey) samples can be brought into contact with R1 and R2 in a laboratory, and from the analysis it can be determined which strains from R1 and R2 have phage relationships with strains from R3. The phage relationship can indicate whether the strains of the bacterial cultures are attacked by the phages in the (whey) samples (cf. sensitivities), which is to be avoided. Furthermore, also (whey) samples can be taken when the rotation of R4 is employed. Similarly, R1 and R2 are exposed to R4 in order to determine the phage relationships between the strains thereof (cf. sensitivities). For example, the initial order R3, R1, R4, R2, may be changed to R3, R2, R4, R1 based on the bacteriophage sensitivities. For instance, R2 may have the least bacteriophage sensitivity overlap (preferably no overlap) compared to R3, i.e. as little as possible bacterial strains that have the same phage sensitivity. The results may indicate that if the current rotation scheme is used, there exists a risk factor for phage infection, and that the adapted order R3, R2, R4, R1 may significantly reduce the risk factor.

The adapted order can be chosen such as to ensure that the risk for bacteriophage infections is minimized. For example, if two strains are present in R1 that have bacteriophage sensitivities in relation to R3, and if only one strain is present in R2 that has bacteriophage sensitivities in relation to R3, then R2 can be placed after R3 in the adapted order.

The process may for instance be carried out by taking samples and performing tests. For instance, whey samples can be taken during the process. By means of experimental verification a data set (e.g. compatibility matrix) can be drawn up, based on which the adapted order of bacterial cultures can be determined. A most distant neighbor can be calculated based on the data set that is available. If the greatest distance has been calculated between the different rotations, it can also be determined which rotations must be carried out one after the other in order to obtain an adapted order providing improved results.

It will be appreciated that an unknown bacterial culture may for instance be a third party product (e.g. another provider). Information with regard to the strains of the other provider's product may be limited or even unavailable. So there is information missing, which is needed in order to determine an adequate culturing order.

It will be appreciated that any of the aspects, features and options described in view of the method apply equally to the process, and the described computer program. It will also be clear that any one or more of the above aspects, features and options can be combined.

### Brief description of the drawings

The invention will further be elucidated on the basis of exemplary embodiments which are represented in a drawing. The exemplary embodiments are given by way of non-limitative illustration. It is noted that the figures are only schematic representations of embodiments of the invention that are given by way of non-limiting example.

In the drawings:
Fig. 1 shows a schematic diagram of an embodiment of a method for determining an order in which a food product is successively cultured during fermentation;
Fig. 2A shows a schematic diagram of acidification and the influence of a phage infection;
Fig. 2B shows a schematic diagram of an embodiment of a method for rotating bacterial cultures in function of phage concentration;
Fig. 3 shows a schematic diagram of an embodiment of a compatibility matrix of bacterial cultures regarding bacteriophage sensitivity; and
Fig. 4 shows a schematic diagram of a method of the invention.

### Detailed description of the invention

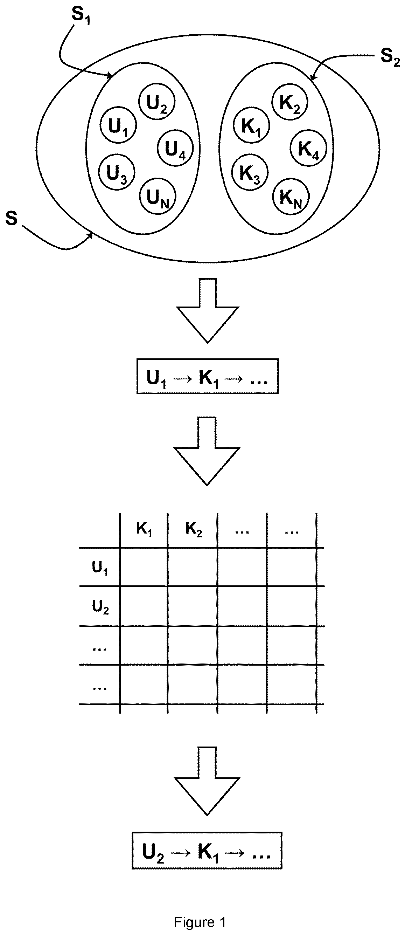

Fig. 1 shows a schematic diagram of an embodiment of a method for determining an order in which a food product is successively cultured during fermentation. The starting point can be found in the top part of the figure, showing a set of bacterial cultures in a process for producing a fermented food product, which are divided in two subsets, a first subset of one or more bacterial cultures (U₁-U_{N}) comprising one or more unknown bacterial strains, and a second subset of one or more bacterial cultures (K₁-K_{N}) comprising one or more known bacterial strains, wherein each of the bacterial strains of the second subset is known. Both subsets are herein shown as containing five bacterial cultures, but both subsets can contain any amount of bacterial cultures, with a minimum of one. Preferably, the total number of bacterial cultures in the set includes two to twenty bacterial cultures, more preferably three to ten bacterial cultures, even more preferably three to six. Each bacterial culture comprises one or more bacterial strains. Preferably, the bacterial cultures do not significantly differ in performance between each other, in order to obtain a continuous quality between batches of fermented food products.

An initial order of bacterial cultures is chosen for rotating during food product fermentation. This can be carried out based on the knowledge of the sensitivity to bacteriophages of the known bacterial strains, and potentially by the incomplete data available for unknown bacterial strains. Alternatively, the initial order of bacterial cultures is randomly distributed. Fig. 1 shows a first bacterial culture with at least one unknown bacterial strain first, subsequently followed by a bacterial culture with known bacterial strains, but this is in no way a requirement.

The food product is then fermented in the presence of the first bacterial culture. Batches may be changed every time a certain pH is reached or a certain time has passed. Multiple subsequent batches can be produced with one bacterial culture before switching to a next bacterial culture. Following fermentation in the presence of the first bacterial culture, the food product is fermented with the subsequently chosen bacterial culture. This process is repeated until each of the bacterial cultures has been used. During or after fermentation with each bacterial culture comprising at least one unknown bacterial strain, a process sample is taken from anything that came into contact with said unknown bacterial culture, e.g. rinsing water, whey, fermented food product, curd, etc.

In a next step, said process samples are each brought into contact with culture samples of the known bacterial cultures and/or cultures of the known bacterial strains and bacteriophages are detected or quantified in the known bacterial cultures and/or cultures of the known bacterial strains. Preferably, the process samples are each brought into contact with each of the bacterial cultures and/or cultures of the known bacterial strains. A compatibility matrix between known and unknown strains can be filled out, as indicated in the third step of fig. 1.

Based on the compatibility matrix, an adapted order of bacterial cultures can be determined, wherein incompatible cultures used successively are minimized, preferably are incompatible cultures not placed subsequently at any point in the rotation. The term "incompatible cultures", as used herein, may refer to bacterial cultures which at least one same bacteriophage can infect, and thus impede growth of said bacterial cultures and therefore harm acidification performance.

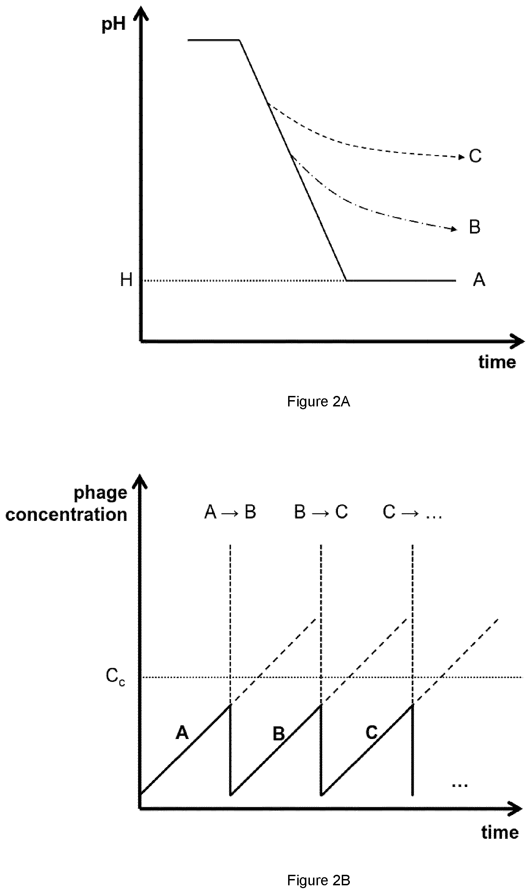

Fig. 2A shows a schematic diagram of acidification and the influence of a phage infection. In a graphical view of the pH in function of the time, an ideal situation would be outcome A. Threshold H is the ideal pH after acidification, which has been reached in situation A, wherein no influence of bacteriophages is detected. In case of a moderate phage infection, the acidification, thus lowering of the pH, will be slowed down and a longer time is necessary to reach threshold pH. This is depicted by outcome B. Lastly, in case of a severe phage infection, outcome C is reached. The acidification is compromised to such a degree, that reaching the threshold pH is unlikely or will take a long time. This diagram signifies the importance of good bacteriophage control and a phage-robust rotation scheme.

Fig. 2B shows a schematic diagram of an embodiment of a method for rotating bacterial cultures in function of phage concentration. When bacterial culture A is used, the concentration of bacteriophages against bacterial culture A will increase, either linear, as depicted in fig. 2B, or non-linear, as function of time and as function of the number of subsequent batches of food product that are produced with bacterial culture A. After a specific time, or a specific pH threshold is reached, bacterial culture A will be replaced by bacterial culture B, as depicted by the vertical dotted line. If bacterial culture B is compatible with bacterial culture A, thus no common bacteriophage sensitivities are present, the bacteriophage concentration is reduced to a low level, at which point different bacteriophages can start growing, which can infect bacterial strains from bacterial culture B. In the case where bacterial culture is incompatible with bacterial culture A, thus common bacteriophage sensitivities exist, the leftmost diagonal dotted line may appear. In this case, the same bacteriophages will be able to multiply further and will reach a critical concentration C_{c}, wherein the acidification with bacterial culture B will no longer be able to reach the desired pH within the target time. The phage concentration growth is here depicted as linear, but may also be non-linear. In the case wherein bacterial culture A and B are compatible, the process can be continued and repeated with bacterial culture C, etc.

Bacteriophages are present everywhere, including bulk starter cultures. Generally, bacterial cultures will be rotated during the process, in the hope that the strains in the cultures are different so no common bacteriophage sensitivities are present.

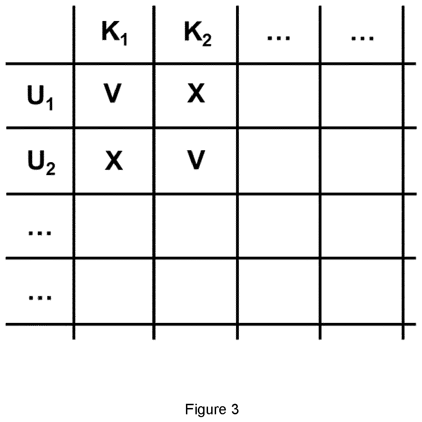

Fig. 3 shows a schematic diagram of an embodiment of a compatibility matrix of bacterial cultures regarding bacteriophage sensitivity. A "V" signifies bacteriophage compatibility, thus no common bacteriophage sensitivities, an "X" signifies bacteriophage incompatibility, thus having common bacteriophage sensitivities. In this example, K₁ and U₁ are compatible and can thus be successive in an adapted order. K₁ and U₂ are incompatible, and it should thus be avoided to put said bacterial cultures successively in an adapter order of a bacteriophage rotation.

In an example, a supplier of bacterial cultures will provide the user with the correct order of his provided bacterial cultures, as this provider would have tested each culture against known bacteriophages and each other. However, whenever a food producer wants to mix bacterial cultures in his rotation of more than one supplier, he does not know the correct rotation order of each of the bacterial cultures, as suppliers usually do not disclose bacteriophage information. If bacterial cultures of different suppliers are used, the user has no way to optimize the order but trial and error, or characterizing each bacterial culture. However, with the above mentioned method, this is no longer necessary. By running the rotation of bacterial cultures just once, analysis can be performed by one of the suppliers on a sample resulting from the bacterial cultures that comprise unknown strains.

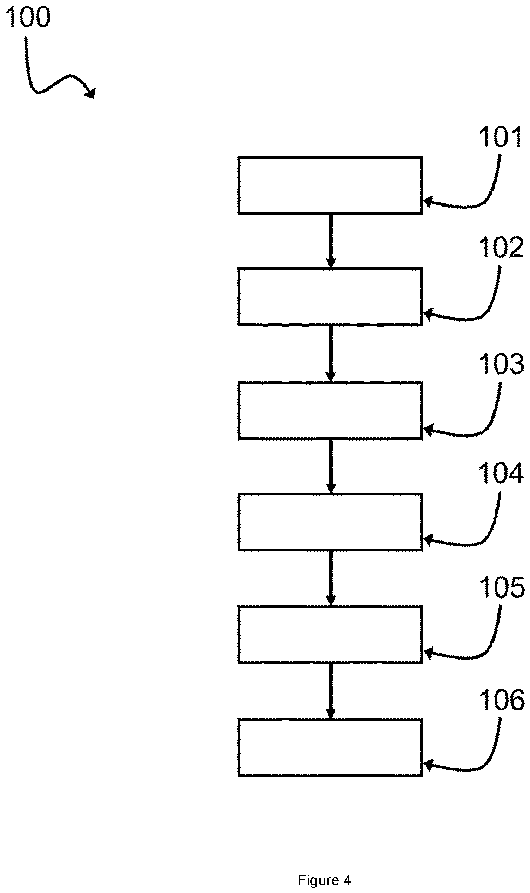

Fig. 4 shows a schematic diagram of a method 100 for determining an order in which a food product is successively cultured with a set of bacterial cultures in a process for producing a fermented food product. In some examples, part of the method is a computer implemented method configured to be run on a machine. In a first step 101, a first subset is defined of one or more bacterial cultures comprising one or more unknown bacterial strain. In a second step 102, a second subset is defined of one or more bacterial cultures comprising one or more known bacterial strains, wherein each of the bacterial strains of the second subset is known. In a third step 103, the process is carried out with an initial order of the set of bacterial cultures. In a fourth step 104, during culturing with each bacterial culture of the first subset, a process sample is collected. In a fifth step 105, a culture sample of a bacterial culture of the second subset is exposed to the collected process samples in order to determine bacteriophage sensitivities of the one or more bacterial cultures of the second subset to bacteriophages present in the collected process sample. In a sixth step 106, an adapted order of the set of bacterial cultures is determined based on the determined bacteriophage sensitivities such as to reduce common bacteriophage sensitivities in successive bacterial cultures.

It will be appreciated that the method may include computer implemented steps. All above mentioned steps can be computer implemented steps. The invention also extends to computer programs, optionally computer programs on or in a carrier, adapted for putting the invention into practice. The program may be in the form of source or object code or in any other form suitable for use in the implementation of the processes according to the invention. The carrier may be any entity or device capable of carrying the program. For example, the carrier may comprise a storage medium, such as a ROM, for example a semiconductor ROM or hard disk. Further, the carrier may be a transmissible carrier such as an electrical or optical signal which may be conveyed via electrical or optical cable or by radio or other means, e.g. via the internet or cloud.

Some embodiments may be implemented, for example, using a machine or tangible computer-readable medium or article which may store an instruction or a set of instructions that, if executed by a machine, may cause the machine to perform a method and/or operations in accordance with the embodiments.

Various embodiments may be implemented using hardware elements, software elements, or a combination of both. Examples of hardware elements may include processors, microprocessors, circuits, application specific integrated circuits (ASIC), programmable logic devices (PLD), digital signal processors (DSP), field programmable gate array (FPGA), logic gates, registers, semiconductor device, microchips, chip sets, et cetera. Examples of software may include software components, programs, applications, computer programs, application programs, system programs, machine programs, operating system software, mobile apps, middleware, firmware, software modules, routines, subroutines, functions, computer implemented methods, procedures, software interfaces, application program interfaces (API), methods, instruction sets, computing code, computer code, et cetera.

Herein, the invention is described with reference to specific examples of embodiments of the invention. It will, however, be evident that various modifications, variations, alternatives and changes may be made therein, without departing from the essence of the invention.

The specifications, figures and examples are, accordingly, to be regarded in an illustrative sense rather than in a restrictive sense. The invention is intended to embrace all alternatives, modifications and variations which fall within the scope of the appended claims. Further, many of the elements that are described are functional entities that may be implemented as discrete or distributed components or in conjunction with other components, in any suitable combination and location.

The word 'comprising' does not exclude the presence of other features or steps than those listed in a claim. Furthermore, the words 'a' and 'an' shall not be construed as limited to 'only one', but instead are used to mean 'at least one', and do not exclude a plurality. The mere fact that certain measures are recited in mutually different claims does not indicate that a combination of these measures cannot be used to an advantage.

## Claims

1. A method for determining an order in which a food product is successively cultured with a set of bacterial cultures in a process for producing a fermented food product, wherein the set of bacterial cultures comprises a first subset of one or more bacterial cultures comprising one or more unknown bacterial strain, and a second subset of one or more bacterial cultures comprising one or more known bacterial strains, wherein each of the bacterial strains of the second subset is known;
wherein the process is carried out with an initial order of the set of bacterial cultures; wherein during culturing with each bacterial culture of the first subset a process sample is collected,
wherein a culture sample of a bacterial culture of the second subset is exposed to the collected process samples in order to determine bacteriophage sensitivities of the one or more bacterial cultures of the second subset to bacteriophages present in the collected process sample,
wherein an adapted order of the set of bacterial cultures is determined based on the determined bacteriophage sensitivities of each of the cultures, such as to reduce common bacteriophage sensitivities in successive bacterial cultures.

2. The method according to claim 1, wherein in a subsequent process the food product is successively cultured with the set of bacterial cultures in the adapted order.

3. The method according to claim 1 or 2, wherein the adapted order is employed dynamically during the process for producing the fermented food product.

4. The method according to any one of the preceding claims, wherein each bacterial strain of the one or more bacterial cultures of the second subset is exposed to the collected process sample for determining the bacteriophage sensitivities.

5. The method according to any one of the preceding claims, wherein one or more bacterial strains of the one or more bacterial cultures of the second subset are individually exposed to each of the collected process samples.

6. The method according to any one of the claims 1-4, wherein combined bacterial strains of the one or more bacterial cultures of the second subset are exposed to each of the collected process samples.

7. The method according to any one of the preceding claims, wherein the adapted order of the set of bacterial cultures is selected such as to minimize common sensitivities to bacteriophages in successive bacterial cultures.

8. The method according to any one of the preceding claims, wherein determining bacteriophage sensitivities is performed by determining a value indicative for the number of bacteriophages in the collected process sample.

9. The method according to claim 8, wherein the value indicative for the number of bacteriophages is determined by performing one or more quantitative polymerase chain reaction (qPCR) measurements.

10. The method according to any one of the preceding claims, wherein the fermented food product is a dairy product, preferably a cheese or a yoghurt.

11. The method according to any of the preceding claims, wherein determining the value indicative for the number of bacteriophages is performed by detecting and/or identifying bacteriophages in the sample of the process for producing a fermented food product.

12. The method according to any one of the preceding claims, wherein information regarding bacteriophage sensitivities are stored in a database, wherein the database is used for determining culturing order in a different process for producing fermented food product.

13. The method according to any one of the preceding claims, wherein the set of bacterial cultures includes two to twenty bacterial cultures, more preferably three to ten bacterial cultures, even more preferably three to six bacterial cultures.

14. The method according to any one of the preceding claims, wherein the one or more bacterial cultures of the first subset are provided by one or more first providers, and wherein the one or more bacterial cultures of the second subset are provided by one or more second providers, wherein the one or more first and second providers are different.

15. A process for producing a fermented food product by successively culturing a food product with a set of bacterial cultures, wherein an order of bacterial cultures in which the food product is successively cultured is determined by performing the method according to any one of the claims 1-14.

16. A computer program product configured to be run on a machine for selecting consecutive bacterial cultures for culturing a food product in a process for producing a fermented food product, the computer program product being configured to perform the method according to any one of the claims 1-14 for determining an order in which the food product is successively cultured with a set of bacterial cultures.

## Patentansprüche

1. Verfahren zum Bestimmen einer Reihenfolge, in der ein Lebensmittelprodukt sukzessiv mit einem Satz von Bakterienkulturen in einem Prozess zum Produzieren eines fermentierten Lebensmittelprodukts kultiviert wird, wobei der Satz der Bakterienkulturen einen ersten Teilsatz von einer oder mehreren Bakterienkulturen, die einen oder mehrere unbekannte Bakterienstämme umfassen, und einen zweiten Teilsatz von einer oder mehreren Bakterienkulturen umfasst, der einen oder mehrere bekannte Bakterienstämme umfasst, wobei jeder der Bakterienstämme des zweiten Teilsatzes bekannt ist;
wobei der Prozess mit einer anfänglichen Reihenfolge des Satzes von Bakterienkulturen durchgeführt wird; wobei während des Kultivierens mit jeder Bakterienkultur des ersten Teilsatzes eine Prozessprobe gesammelt wird,
wobei eine Kulturprobe einer Bakterienkultur des zweiten Teilsatzes den gesammelten Prozessproben ausgesetzt wird, um Bakteriophagenempfindlichkeiten der einen oder mehreren Bakterienkulturen des zweiten Teilsatzes auf Bakteriophagen zu bestimmen, die in der gesammelten Prozessprobe vorhanden sind,
wobei basierend auf den bestimmten Bakteriophagenempfindlichkeiten von jeder der Kulturen eine adaptierte Reihenfolge des Satzes von Bakterienkulturen bestimmt wird, um so gemeinsame Bakteriophagenempfindlichkeiten in sukzessiven Bakterienkulturen zu reduzieren.

2. Verfahren nach Anspruch 1, wobei das Lebensmittelprodukt in einem nachfolgenden Prozess sukzessiv mit dem Satz der Bakterienkulturen in der adaptierten Reihenfolge kultiviert wird.

3. Verfahren nach Anspruch 1 oder 2, wobei die adaptierte Reihenfolge dynamisch während des Prozesses zum Produzieren des fermentierten Lebensmittelprodukts verwendet wird.

4. Verfahren nach einem der vorhergehenden Ansprüche, wobei jeder Bakterienstamm der einen oder den mehreren Bakterienkulturen des zweiten Teilsatzes der gesammelten Prozessprobe ausgesetzt wird, um die Bakteriophagenempfindlichkeiten zu bestimmen.

5. Verfahren nach einem der vorhergehenden Ansprüche, wobei ein oder mehrere Bakterienstämme der einen oder mehreren Bakterienkulturen des zweiten Teilsatzes individuell jeder der gesammelten Prozessproben ausgesetzt wird bzw. werden.

6. Verfahren nach einem der Ansprüche 1 bis 4, wobei kombinierte Bakterienstämme der einen oder mehreren Bakterienkulturen des zweiten Teilsatzes jeder der gesammelten Prozessproben ausgesetzt werden.

7. Verfahren nach einem der vorhergehenden Ansprüche, wobei die adaptierte Reihenfolge des Satzes von Bakterienkulturen so gewählt wird, dass gemeinsame Empfindlichkeiten gegen Bakteriophagen in sukzessiven Bakterienkulturen minimiert werden.

8. Verfahren nach einem der vorhergehenden Ansprüche, wobei Bestimmen von Bakteriophagenempfindlichkeiten durchgeführt wird, indem ein Wert bestimmt wird, der ein Indikator für die Anzahl der Bakteriophagen in der gesammelten Prozessprobe ist.

9. Verfahren nach Anspruch 8, wobei der Wert, welcher der Indikator für die Anzahl der Bakteriophagen ist, bestimmt wird, indem eine oder mehrere quantitative Polymerasekettenreaktions- (qPCR)-Messungen durchgeführt wird bzw. werden.

10. Verfahren nach einem der vorhergehenden Ansprüche, wobei das fermentierte Lebensmittelprodukt ein Milchprodukt, vorzugsweise ein Käse oder ein Joghurt, ist.

11. Verfahren nach einem der vorhergehenden Ansprüche, wobei Bestimmen des Werts, der ein Indikator für die Anzahl der Bakteriophagen ist, durch Detektieren und/oder Identifizieren von Bakteriophagen in der Probe des Prozesses zur Produktion eines fermentierten Lebensmittelprodukts durchgeführt wird.

12. Verfahren nach einem der vorhergehenden Ansprüche, wobei Informationen hinsichtlich der Bakteriophagenempfindlichkeiten in einer Datenbank gespeichert werden, wobei die Datenbank zum Bestimmen der Kultivierungsreihenfolge in einem anderen Prozess zum Produzieren von fermentiertem Lebensmittelprodukt verwendet wird.

13. Verfahren nach eine der vorhergehenden Ansprüche, wobei der Satz der Bakterienkulturen zwei bis zwanzig Bakterienkulturen, vorzugsweise drei bis zehn Bakterienkulturen, noch bevorzugter drei bis sechs Bakterienkulturen einschließt.

14. Verfahren nach einem der vorhergehenden Ansprüche, wobei die eine oder mehreren Bakterienkulturen des ersten Teilsatzes durch einen oder mehrere erste Anbieter bereitgestellt wird bzw. werden, und wobei die eine oder mehreren Bakterienkulturen des zweiten Teilsatzes durch einen oder mehrere zweite Anbieter bereitgestellt wird bzw. werden, wobei der eine oder die mehreren ersten und zweiten Anbieter sich unterscheiden.

15. Prozess zum Produzieren eines fermentierten Lebensmittelprodukts durch sukzessives Kultivieren eines Lebensmittelprodukts mit einem Satz von Bakterienkulturen, wobei eine Reihenfolge der Bakterienkulturen, in der das Lebensmittelprodukt sukzessiv kultiviert wird, mittels Durchführen des Verfahrens gemäß einem der Ansprüche 1 bis 14 bestimmt wird.

16. Computerprogrammprodukt, das ausgestaltet ist, um auf einer Maschine zum Auswählen von aufeinander folgenden Bakterienkulturen zum Kultivieren eines Lebensmittelprodukts in einem Prozess zum Produzieren eines fermentierten Lebensmittelprodukts ausgeführt zu werden, wobei das Computerprogrammprodukt ausgestaltet ist, um das Verfahren gemäß einem der Ansprüche 1 bis 14 durchzuführen, um eine Reihenfolge zu bestimmen, in der das Lebensmittelprodukt sukzessiv mit einem Satz von Bakterienkulturen kultiviert wird.

## Revendications

1. Procédé pour déterminer un ordre dans lequel un produit alimentaire est successivement mis en culture avec un ensemble de cultures bactériennes dans un processus de production d'un produit alimentaire fermenté, l'ensemble de cultures bactériennes comprenant un premier sous-ensemble d'une ou plusieurs cultures bactériennes comprenant une ou plusieurs souches bactériennes inconnues, et un second sous-ensemble d'une ou plusieurs cultures bactériennes comprenant une ou plusieurs souches bactériennes connues, chacune des souches bactériennes du second sous-ensemble étant connue ; le procédé étant mis en œuvre avec un ordre initial de l'ensemble de cultures bactériennes ;
au cours de la culture de chaque culture bactérienne du premier sous-ensemble, un échantillon de traitement étant collecté,
un échantillon de culture d'une culture bactérienne du second sous-ensemble étant exposé aux échantillons de traitement collectés afin de déterminer des sensibilités aux bactériophages de la ou des cultures bactériennes du second sous-ensemble aux bactériophages présents dans l'échantillon de traitement collecté,
un ordre adapté de l'ensemble de cultures bactériennes étant déterminé sur la base des sensibilités aux bactériophages déterminées pour chacune des cultures, de manière à réduire des sensibilités aux bactériophages communes dans des cultures bactériennes successives.

2. Procédé selon la revendication 1, dans un procédé ultérieur, le produit alimentaire étant successivement mis en culture avec l'ensemble de cultures bactériennes dans l'ordre adapté.

3. Procédé selon la revendication 1 ou 2, l'ordre adapté étant utilisé de manière dynamique au cours du processus de production du produit alimentaire fermenté.

4. Procédé selon l'une quelconque des revendications précédentes, chaque souche bactérienne de la ou des cultures bactériennes du second sous-ensemble étant exposée à l'échantillon de traitement collecté pour déterminer les sensibilités aux bactériophages.

5. Procédé selon l'une quelconque des revendications précédentes, une ou plusieurs souches bactériennes de la ou des cultures bactériennes du second sous-ensemble étant exposées individuellement à chacun des échantillons de traitement collectés.

6. Procédé selon l'une quelconque des revendications 1 à 4, des souches bactériennes combinées de la ou des cultures bactériennes du second sous-ensemble étant exposées à chacun des échantillons de traitement collectés.

7. Procédé selon l'une quelconque des revendications précédentes, l'ordre adapté de l'ensemble de cultures bactériennes étant choisi de manière à minimiser des sensibilités aux bactériophages communes dans des cultures bactériennes successives.

8. Procédé selon l'une quelconque des revendications précédentes, la détermination des sensibilités aux bactériophages étant réalisée par détermination d'une valeur indicative du nombre de bactériophages dans l'échantillon de traitement collecté.

9. Procédé selon la revendication 8, la valeur indicative du nombre de bactériophages étant déterminée par réalisation d'une ou plusieurs mesures quantitatives de réaction en chaîne par polymérase (qPCR).

10. Procédé selon l'une quelconque des revendications précédentes, le produit alimentaire fermenté étant un produit laitier, de préférence un fromage ou un yaourt.

11. Procédé selon l'une quelconque des revendications précédentes, la détermination de la valeur indicative du nombre de bactériophages étant réalisée par la détection et/ou l'identification de bactériophages dans l'échantillon du processus de production d'un produit alimentaire fermenté.

12. Procédé selon l'une quelconque des revendications précédentes, des informations concernant les sensibilités aux bactériophages étant stockées dans une base de données, la base de données étant utilisée pour déterminer l'ordre de culture dans un processus de production d'un produit alimentaire fermenté différent.

13. Procédé selon l'une quelconque des revendications précédentes, l'ensemble de cultures bactériennes comprenant de deux à vingt cultures bactériennes, de préférence de trois à dix cultures bactériennes, de préférence encore de trois à six cultures bactériennes.

14. Procédé selon l'une quelconque des revendications précédentes, la ou les cultures bactériennes du premier sous-ensemble étant fournies par un ou plusieurs premiers fournisseurs, et la ou les cultures bactériennes du second sous-ensemble étant fournies par un ou plusieurs seconds fournisseurs, le ou les premier et second fournisseurs étant différents.

15. Processus de production d'un produit alimentaire fermenté par mise en culture successive d'un produit alimentaire avec un ensemble de cultures bactériennes, un ordre des cultures bactériennes dans lequel le produit alimentaire est successivement mis en culture étant déterminé par la réalisation du procédé selon l'une quelconque des revendications 1 à 14.

16. Programme informatique configuré pour être exécuté sur une machine afin de sélectionner des cultures bactériennes consécutives pour mettre en culture un produit alimentaire dans un processus de production d'un produit alimentaire fermenté, le programme informatique étant configuré pour réaliser le procédé selon l'une quelconque des revendications 1 à 14 afin de déterminer un ordre dans lequel le produit alimentaire est successivement mis en culture avec un ensemble de cultures bactériennes.
